Europäisches Patentamt

European Patent Office

Office européen des brevets

(19)

(11) Veröffentlichungsnummer: **0 395 950**

**A1**

(12) **EUROPÄISCHE PATENTANMELDUNG**

(21) Anmeldenummer: 90107495.5

(22) Anmeldetag: 20.04.90

(51) Int. Cl.⁵: **C12N 15/02, //C12N15/05**

(30) Priorität: 29.04.89 DE 3914339

(43) Veröffentlichungstag der Anmeldung:
07.11.90 Patentblatt 90/45

(84) Benannte Vertragsstaaten:
**AT BE CH DE DK ES FR GB GR IT LI LU NL SE**

(71) Anmelder: **B. Braun Melsungen AG**
**Carl-Braun Strasse**
**D-3508 Melsungen(DE)**

(72) Erfinder: **Zimmermann, Ulrich, Prof.-Dr.**
**Pfarrer-Fröhlichstrasse 17**
**D-8702 Waldbrunn(DE)**
Erfinder: **Klöck, Gerd, Dr.**
**Sudetenstrasse 9a**
**D-8702 Zell am Main(DE)**
Erfinder: **Gessner, Petra**
**Hauptstrasse 10**
**D-8701 Riedenheim(DE)**

(74) Vertreter: **LOUIS, PÖHLAU, LOHRENTZ &**
**SEGETH**
**Kesslerplatz 1 Postfach 3055**
**D-8500 Nürnberg-20(DE)**

(54) Verfahren zur Elektro-Fusion von Zellen unter Verwendung eines elektrischen Wechselfeldes.

(57) Es wird ein Verfahren zur Elektro-Fusion von Zellen vorgeschlagen, bei dem die Zellen in üblicher Weise mittels Dielektrophorese in Kontakt miteinander gebracht und dann mittels wenigstens eines Gleichspannungsimpulses die Zellmembranen benachbarter Zellen zwecks Zellfusion durchbrochen werden. Wesentlich bei dem Verfahren ist, dass das für die Dielektrophorese angelegte Wechselfeld zuerst höhere Feldstärke aufweist, diese Feldstärke dann rampenartig auf eine niedrigere Feldstärke reduziert wird und dass schliesslich die niedrigere Feldstärke für die gewünschte Kontaktierungszeit der Zellmembranen aufrechterhalten wird, wobei der wenigstens eine Gleichspannungsimpulse erst nach Erreichen der niedrigeren Feldstärke angelegt wird.

## Verfahren zur Elektro-Fusion von Zellen unter Verwendung eines elektrischen Wechselfeldes

Die Erfindung bezieht sich auf ein Verfahren zur Elektro-Fusion von Zellen, bei welchem die Zellen, gegebenenfalls nach vorheriger Waschbehandlung, in einer Flüssigkeit einem ungleichförmigen elektrischen Wechselfeld üblicher Feldstärke zwecks gegenseitiger Annäherung der Zellen ausgesetzt und nach Anlagerung von Zellen aneinander mittels wenigstens eines Gleichspannungsimpulses die Zellmembranen benachbarter Zellen durchbrochen werden, um so eine Zellfusion zu bewirken. Das Verfahren nach der Erfindung kann auf unterschiedlichste Zellen angewendet werden, z.B. auf tierische Zellen, Pflanzenzellen bzw. zumindest Pflanzen-Protoplasten, Hefen und Bakterien.

Beispielsweise in dem Artikel "BIOPHYSICS OF ELECTROINJECTION AND ELECTROFUSION" von Zimmermann und Arnold im "Journal of Electrostatics", Band 21 (1988), Seite 309 - 345, sind die Grundsätze der Zellmodifikation durch Elektro-Injection und Elektro-Fusion beschrieben, wobei auch dargelegt ist, welche Verfahren für die Elektro-Fusion zur Verfügung stehen. Unter anderem ist dort auch das gattungsgemässe Verfahren beschrieben, bei dem aufgrund der sogenannten Dielektrophorese Zellen mit ihren Membranen in Kontakt gebracht und dann mittels eines Gleichspannungsimpulses sehr kurzer Dauer die Membranen im Berührungsbereich durchstossen werden, wodurch es zu einer Zellfusion kommt. Als Dielektrophorese bezeichnet man dabei die Bewegung von Zellen aufeinander zu in einem ungleichförmigen, elektrischen Wechselfeld, wobei sich die Zellen abhängig von ihren Eigenschaften beispielsweise in Bereichen besonders hoher oder besonders niedriger Feldstärke sammeln können.

Bisher geht man bei der Zellfusion grundsätzlich so vor, dass zumindest bis zur Perforation der Zellmembran ein elektrisches Wechselfeld mit praktisch konstanter Feldstärke angelegt wird. Ein derartiges Vorgehen hat jedoch gewisse Nachteile. Zum einen haben Versuche gezeigt, dass bei Anlegen eines Wechselfeldes konstanter Feldstärke sich jeweils nur Zellen eines bestimmten, sehr ähnlichen Grössenbereiches aneinander anlagern, während Zellen anderer Grösse nicht zur Anlagerung an diese Zellen gelangen. Dies hat zur Folge, dass bei der Fusion dann auch jeweils nur in etwa gleich grosse Zellen miteinander verschmolzen werden, so dass die modifizierten Zellen jeweils in sich in etwa gleiche Grösse haben. Dies ist unerwünscht, weil ja auch die Zellen des Ausgangsmediums unterschiedliche Grösse aufweisen. Man ist deswegen daran interessiert, ein Verfahren zu entwickeln, das zur Fusion unterschiedlich grosser Zellen im wesentlichen entsprechend dem ursprünglichen Anteil dieser Zellen führt.

Ein weiteres Problem der Dielektrophorese besteht darin, dass manche Zellen sehr empfindlich gegen das angelegte Wechselfeld sind. Wenn die Feldstärke zu gross wird, besteht die Gefahr, dass Zellwände bereits verhältnismässig bald geschädigt werden. Wenn dann das störende Feld längere Zeit anliegt, können irreversible Schädigungen der Zellen die Folge sein. Man muss deswegen bei den bisher üblichen Verfahren die Feldstärke des für die Kontaktierung der Zellen verwendeten Wechselfeldes sehr genau bestimmen.

Der Erfindung liegt nun die Aufgabe zugrunde, ein Verfahren für die Elektro-Fusion vorzuschlagen, welches es gestattet, die vorstehend erwähnten Schwierigkeiten auszuräumen, d.h. einerseits die Möglichkeit bietet, bei der Elektro-Fusion auch Zellen unterschiedlicher Grösse in erheblichem Umfange zu verschmelzen und andererseits es möglich macht, unter Bedingungen zu arbeiten, bei denen eine Schädigung von Zellen vor Anlegen des Perforations-Gleichspannungsimpulses bzw. der Gleichspannungsimpulse nicht zu erwarten ist, auch wenn die Feldstärke des für die Dielektrophorese verwendeten Wechselfeldes nicht mit sehr grosser Genauigkeit eingestellt wird.

Zur Lösung dieser Aufgabe wird erfindungsgemäss bei einem Verfahren der eingangs erwähnten Art nun vorgeschlagen, dass zuerst ein Wechselfeld höherer Felstärke angelegt wird, dass hierauf die höhere Feldstärke rampenartig, d.h. im wesentlichen gleichmässig bzw. kontinuierlich, auf eine niedrigere Feldstärke reduziert wird, dass dann die niedrigere Feldstärke für die gewünschte Kontaktierungszeit der Zellmembranen aufrechterhalten wird, und dass der wenigstens eine Gleichspannungsimpuls nach Erreichen der niedrigeren Feldstärke, d.h. entweder solange die niedrigere Felstärke noch anliegt oder anschliessend an das elektrische Wechselfeld niedrigerer Felstärke, angelegt wird.

Entgegen dem bisherigen Vorgehen wird also erfindungsgemäss bei der Dielektrophorese kein konstantes Wechselfeld angelegt sondern zuerst mit einem höhereren Wechselfeld und dann mit einem allmählich eingestellten niedrigeren Wechselfeld gearbeitet. Abhängig von den Verfahrensbedingungen erreicht man dabei einerseits eine Anlagerung von Zellen unterschiedlicher Grösse oder es wird andererseits dafür gesorgt, dass auch dann, wenn die ursprüngliche Feldstärke relativ hoch ist, es nicht zu vorzeitigen Schädigungen der Zellen kommt.

Dabei kann man erfindungsgemäss zwei Wege wählen -:

Wenn man erreichen will, dass im wesentlichen keine vorzeitige Schädigung der Zellen eintritt, geht man so vor, dass das Wechselfeld höherer Feldstärke etwa der für die Anlagerung von Zellen zwecks Zellfusion verwendeten üblichen Feldstärke entspricht, und dass die niedrigere Feldstärke dann niedriger als die Hälfte der höheren Feldstärke ist. Dabei kann man durchaus mit einer höhereren Feldstärke arbeiten, die etwas grösser ist als die sonst übliche Feldstärke, weil infolge der vergleichweise kurzen Einwirkungszeit der höheren Feldstärke es nicht zu Beschädigungen der Zellmembran kommt.

Will man dagegen erreichen, dass bei der Fusion nicht nur in etwa gleich grosse Zellen sondern in erheblichem Umfange auch Zellen unterschiedlicher Grösse miteinander verschmelzen, d.h. durch die Dielektrophorese in Kontakt miteinander kommen, ist erfindungsgemäss vorgesehen, dass die höhere Feldstärke wenigstens doppelt so gross wie die übliche Feldstärke ist und die niedrigere Feldstärke etwa der üblichen Feldstärke entspricht. Voraussetzung für eine derartige Vorgehensweise ist natürlich, dass auch bei der höheren Feldstärke keine allzu starken Schädigungen der Zellmembran vor dem eigentlichen Perforations-Gleichspannungsimpuls erfolgen. Es genügt allerdings in einer Vielzahl der Fälle, wenn nur ein Teil der eingesetzten Zellen tatsächlich verschmolzen wird, so dass es grundsätzlich nicht schadet, wenn etliche der Zellen durch die höhere Feldstärke vor der Fusionierung geschädigt werden. Diese Zellen können ja in geeigneter Weise dann abgetrennt werden.

Auch die Geschwindigkeit der rampenartigen Verringerung der höheren Feldstärke auf die niedrigere Feldstärke ist von Bedeutung. Hier wird nach der Erfindung vorgeschlagen, dass die rampenartige Verringerung der höheren Feldstärke auf die niedrigere Feldstärke in einem Zeitraum von 5 bis 30 sec, vorzugsweise von etwa 10 sec, erfolgt.

Das Wechselfeld niedrigerer Feldstärke kann verständlicherweise, da sein Einfluss auf die Zellmembran weniger schädigend ist, länger anliegen als das Wechselfeld höherer Feldstärke. Nach der Erfindung ist nun vorgesehen, dass das Wechselfeld niedrigerer Feldstärke zwischen Verringerung der Feldstärke und erstem Gleichspannungsimpuls, d.h. für die gewünschte Kontaktierungszeit der Zellmembranen, etwa 5 bis 60 sec aufrecht erhalten wird.

Grundsätzlich müssen die jeweiligen Verfahrensbedingungen abhängig vom Einzelfall, d.h. abhängig von den zu fusionierenden Zellen, dem Medium, in dem die Fusionierung erfolgen soll, der Form des Feldes etc., bestimmt werden. Es ist jedoch davon auszugehen, dass bei der Fusionierung von Zellen unterschiedlicher Grösse zweckmässigerweise als niedrigere Feldstärke des Wechselfeldes eine Feldstärke von etwa 100 V/cm verwendet wird, während die höhere Feldstärke wenigstens 300 V/cm beträgt.

Um ungewollte Schädigungen der Zellen vor Anlegung des eigentlichen Perforationsimpulses zu verhindern, ist es günstig, wenn die Spitzenspannung einer Polarität des Wechselfeldes höherer Feldstärke höchstens gleich der halben Maximalspannung des Gleichspannungsimpulses ist.

Um ungünstige Einflüsse auf die Fusion auszuschalten, wird nach der Erfindung vorgeschlagen, die Frequenz des Wechselfeldes niedrigerer und höherer Feldstärke gleich zu wählen. Vorzugsweise liegt die Frequenz des Wechselfeldes niedrigerer und/oder höherer Feldstärke unter 20 MHz, zweckmässigerweise bei 1 bis 4 MHz.

Es wurde bereits oben darauf hingewiesen, dass Wechselfelder höherer Feldstärke zu einer Schädigung der Membran der Zellen vor dem eigentlichen Fusionsvorgang führen können. Um diese Gefahr möglichst gering zu halten, sollte das Wechselfeld höherer Feldstärke vorteilhafterweise höchstens 30 sec, vorzugsweise etwa 10 sec, angelegt werden.

Für die Membran-Perforation können im Rahmen der Erfindung Gleichspannungsimpulse üblicher Dauer und Feldstärke verwendet werden, wobei günstigerweise Gleichspannungsimpulse verwendet werden mit einer Feldstärke von unter 6.000 V/cm, vorzugsweise etwa 1.000 V/cm, und einer Dauer von wenigstens 10 $\mu$sec. Hierbei wird eine besondere zuverlässige Perforation einer grösseren Zahl von Zellen und dabei eine gute Fusions-Ausbeute erreicht, wenn zwei etwa gleiche Gleichspannungsimpulse in einem Abstand zwischen 0,1 und 10 sec angelegt werden, wobei vorteilhafterweise zwischen den Gleichspannungsimpulsen und gegebenenfalls auch kurzzeitig danach das Wechselfeld niedrigerer Feldstärke angelegt bleibt.

Schliesslich liegt es im Rahmen der Erfindung, dass die Zellen vor der Elektro-Behandlung in einer gegenüber den Zellen hypotonen Flüssigkeit gewaschen werden, während die Elektro-Behandlung in einer isotonen Flüssigkeit stattfindet. Ein derartiges Vorgehen hat den Vorteil, dass die Ausbeute an fusionierten Zellen steigt, weil durch die Verwendung eines hypotonen Mediums zum Waschen die Zellen auf die Perforation sehr gut vorbereitet werden. Andererseits wird durch die anschliessende Einbringung in das isotone Medium erreicht, dass die Zellenmembran durch die einwirkenden elektrischen Kräfte nicht so leicht geschädigt werden kann.

Weitere Merkmale, Einzelheiten und Vorteile der Erfindung ergeben sich aus der folgenden Beschreibung von Ausführungsbeispielen der Zellfusion unter Verwendung des erfindungsgemässen Verfahrens.

### Beispiel 1

Es handelt sich um ein Beispiel zur Erläuterung des Vorgehens nach dem Stand der Technik.

Tabakzellen (Nicotiana tabacum) wurden enzymatisch behandelt, um die Zellwand zu entfernen. Anschliessend wurden die so behandelten Protoplasten in einer Konzentration von $10^4$ Protoplasten / ml in 0,6-molares Mannit aufgenommen und in eine geeignete, übliche Fusionskammer gefüllt. In dieser Fusionskammer wurde ein Wechselfeld der Frequenz 900 kHz mit einer für derartige Fälle üblichen Feldstärke von 200 V/cm für 20 sec angelegt, um so die Bildung von Zellketten bzw. Zellpaaren zu erreichen. Zur Fusion wurde dann an die Elektroden der Fusionskammer ein Gleichstromimpuls von 50 $\mu$sec Dauer und mit einer Feldstärke von 1.200 bis 1.500 V/cm angelegt. Hieran anschliessend, d.h. nach der Fusion, wurde das ursprüngliche Wechselfeld von 200 V/cm noch für 60 sec aufrechterhalten, um die gebildeten Zellketten bzw. Zellpaare zusammenzuhalten und die Fusion zu fördern.

### Beispiel 2

Es handelt sich hier um ein Beispiel, wie bei der Fusion von Zellen unterschiedlicher Grösse vorgegangen werden kann, um wieder Zellen unterschiedlicher Grösse zu erhalten.

Zellen von Hafer (Avena sativa) wurden enzymatisch behandelt, um die Zellwand zu entfernen. Anschliessend wurden die Protoplasten in einer Konzentration von $10^4$ Protoplasten / ml in ein übliches Fusionsmedium aufgenommen und in eine Fusionskammer üblicher Ausbildung gefüllt. Es wurde dann ein Wechselfeld mit einer Frequenz von etwa 1 MHz angelegt, wobei die Feldstärke des Wechselfeldes zuerst der höheren Feldstärke entsprechend auf etwa 350 bis 400 V/cm eingestellt wurde. Nach etwa 10 sec wurde dann dieses Wechselfeld kontinuierlich in einer Zeit von ebenfalls etwa 10 sec auf eine niedrigere Feldstärke von etwa 90 V/cm reduziert. Diese niedrigere Feldstärke blieb dann etwa 30 sec angelegt.

Zur Fusion wurden einer oder mehrere Gleichstromimpulse von etwa 1.000 V/cm mit einer Dauer zwischen 10 und 100 $\mu$sec, bevorzugt zwei Pulse von jeweils 20 $\mu$sec Dauer, in die Fusionskammer eingeleitet bzw. an die entsprechenden Elektroden angelegt. Bei Verwendung mehrfacher Pulse betrug dabei der Abstand zwischen den Pulsen bevorzugt 0,5 sec, wobei jedoch auch Abstände zwischen 0,1 und 10 sec zur Anwendung kamen. Nach der Fusion wurde, um die Zellen in Anlage aneinander zu halten, das Wechselfeld niedrigerer Feldstärke für etwa 30 sec aufrechterhalten, um so den Abschluss des Fusionsvorganges zu fördern.

Man erhielt fusionierte Zellen, die im Gegensatz zu einem Vorgehen nach dem Stand der Technik aus Zellen unterschiedlicher Grösse entstanden waren.

Zu dem vorstehenden Beispiel ist noch darauf hinzuweisen, dass selbstverständlich die Feldstärken und Einwirkungsdauer variiert werden können. Die höhere Feldstärke kann nicht nur 10 sondern zwischen etwa 5 und 30 sec angelegt werden können. Der Zeitraum für die rampenartige Verminderung der höheren Feldstärke auf die niedere Feldstärke kann zwischen 5 und 30 sec betragen. Auch hinsichtlich der niedrigeren Feldstärke sind durchaus Variationsmöglichkeiten, beispielsweise zwischen 5 und 200 V/cm gegeben, wobei auch die Einwirkungsdauer der niedrigeren Feldstärke vor der Fusion zwischen 5 und 60 sec betragen kann.

### Beispiel 3

Dieses Beispiel dient zur Erläuterung der Anwendung des Verfahrens nach der Erfindung bei der Fusion von gleich grossen Protoplasten.

Zellen von Hafer (Avena sativa) wurden enzymatisch behandelt, um die Zellwand zu entfernen. Anschliessend wurden die Protoplasten in einer Konzentration von $10^4$ Protoplasten / ml in ein übliches Fusionsmedium aufgenommen und in eine Fusionskammer üblicher Ausbildung gefüllt.

In dieser Fusionskammer wurde ein elektrisches Wechselfeld mit einer Frequenz von 1 MHz angelegt, wobei die Feldstärke zunächst auf einen Wert von 200 V/cm als höhere Feldstärke eingestellt wurde, wobei dieser Wert durchaus einer üblichen Feldstärke entspricht, wie Beispiel 1 zeigt. Nach etwa 10 sec wurde dieses Wechselfeld dann kontinuierlich über einen Zeitraum von ebenfalls 10 sec auf eine niedrigere Feldstärke von 90 V/cm reduziert. Diese niedrigere Feldstärke blieb 30 sec angelegt. Hieran anschliessend wurde dann die Fusion durch Anlegen zweier Gleichstromimpulse einer Feldstärke von 900 V/cm und einer Dauer von 20 $\mu$sec eingeleitet, wobei der Abstand zwischen diesen beiden Pulsen 0,5 sec betrug.

Nach der Fusion wurde dann das Wechselfeld niedrigerer Feldstärke noch weitere 30 sec angelegt, um den Abschluss des Fusionsvorganges zu fördern.

Bei einem derartigen Vorgehen ist es möglich, im wesentlichen nur Zellen gleicher Grösse mitein-

ander zu fusionieren.

**Ansprüche**

1. Verfahren zur Elektro-Fusion von Zellen, bei welchem die Zellen, gegebenenfalls nach vorheriger Waschbehandlung, in einer Flüssigkeit einem ungleichförmigen elektrischen Wechselfeld üblicher Feldstärke zwecks gegenseitiger Annäherung der Zellen ausgesetzt und nach Anlagerung von Zellen aneinander mittels wenigstens eines Gleichspannungsimpulses die Zellmembranen benachbarter Zellen durchbrochen weden, um so eine Zellfusion zu bewirken,
**dadurch gekennzeichnet ,**
dass zuerst ein Wechselfeld höherer Feldstärke angelegt wird, dass hierauf die höhere Feldstärke rampenartig auf eine niedrigere Feldstärke reduziert wird, wobei entweder ein Wechselfeld höherer Feldstärke angelegt wird, das der für die Anlagerung von Zellen zwecks Zellfusion verwendeten üblichen Feldstärke entspricht, und die niedrigere Feldstärke niedriger als die Hälfte der höheren Feldstärke ist, oder wobei die höhere Feldstärke wenigstens doppelt so gross wie die übliche Feldstärke ist, und die niedrigere Feldstärke der üblichen Feldstärke entspricht, und wobei weiter die rampenartige Verringerung der höheren Feldstärke auf die niedrigere Feldstärke in einem Zeitraum von 5 bis 30 sec erfolgt, dass dann die niedrigere Feldstärke für die gewünschte Kontaktierungszeit der Zellmembranen von 5 bis 60 sec aufrechterhalten wird, und dass der wenigstens eine Gleichspannungsimpuls nach Erreichen der niedrigeren Feldstärke angelegt wird.

2. Verfahren nach Anspruch 1,
**dadurch gekennzeichnet ,**
dass als niedrigere, der üblichen entsprechende Feldstärke des Wechselfeldes eine Feldstärke von etwa 100 V/cm verwendet wird und die höhere Feldstärke wenigstens 300 V/cm beträgt.

3. Verfahren nach einem der vorhergehenden Ansprüche, **dadurch gekennzeichnet ,**
dass die Spitzenspannung einer Polarität des Wechselfeldes höherer Feldstärke höchstens gleich der halben Maximalspannung des Gleichspannungsimpulses ist.

4. Verfahren nach einem der vorhergehenden Ansprüche, **dadurch gekennzeichnet ,**
dass die Frequenz des Wechselfeldes niedrigerer und höherer Feldstärke gleich ist.

5. Verfahren nach einem der vorhergehenden Ansprüche, **dadurch gekennzeichnet ,**
dass die Frequenz des Wechselfeldes niedrigerer und/oder höherer Feldstärke unter 20 MHz liegt, vorzugsweise 1 bis 4 MHz beträgt.

6. Verfahren nach einem der vorhergehenden Ansprüche,
**dadurch gekennzeichnet ,**
dass das Wechselfeld höherer Feldstärke höchstens 30 sec, vorzugsweise etwa 10 sec, angelegt wird.

7. Verfahren nach einem der vorhergehenden Ansprüche,
**dadurch gekennzeichnet ,**
dass die rampenartige Verringerung der höheren Feldstärke auf die niedrigere Feldstärke in einem Zeitraum von etwa 10 sec erfolgt.

8. Verfahren nach einem der vorhergehenden Ansprüche,
**dadurch gekennzeichnet ,**
dass zur Membran-Perforation wenigstens ein Gleichspannungsimpuls einer Feldstärke von unter 6.000 V/cm und einer Dauer von wenigstens 10 μsec verwendet wird.

9. Verfahren nach einem der vorhergehenden Ansprüche,
**dadurch gekennzeichnet ,**
dass zwei etwa gleiche Gleichspannungsimpulse in einem Abstand von zwischen 0,1 und 10 sec angelegt werden.

10. Verfahren nach Anspruch 9, **dadurch gekennzeichnet ,**
dass zwischen den Gleichspannungsimpulsen und gegebenenfalls auch kurzzeitig danach das Wechselfeld niedrigerer Feldstärke angelegt bleibt.

11. Verfahren nach einem der vorhergehenden Ansprüche, **dadurch gekennzeichnet ,**
dass die Zellen vor der Elektro-Behandlung in einer gegenüber den Zellen hypotonen Flüssigkeit gewaschen werden, während die Elektro-Behandlung in einer isotonen Flüssigkeit stattfindet.

Europäisches Patentamt

**EUROPÄISCHER RECHERCHENBERICHT**

Nummer der Anmeldung

EP 90 10 7495

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | TRENDS IN BIOTECHNOLOGY, Band 6, Nr. 7, Juli 1988, pages 153-158, Elsevier Publications, Cambridge, GB; M. JONES: "Fusing plant protoplasts"<br>--- | | C 12 N 15/02 //<br>C 12 N 15/05 |
| A | EP-A-0 263 017 (MITSUBISHI CHEM. IND.)<br>----- | | |

|  |  |
|---|---|
| | **RECHERCHIERTE SACHGEBIETE (Int. Cl.5)**<br><br>C 12 N |

Der vorliegende Recherchenbericht wurde für alle Patentansprüche erstellt

| Recherchenort | Abschlußdatum der Recherche | Prüfer |
|---|---|---|
| DEN HAAG | 23-07-1990 | DESCAMPS J.A. |